# EUROPEAN PATENT SPECIFICATION

(11) **EP 2 037 887 B1**
(45) Date of publication and mention of the grant of the patent: **15.09.2010**
(21) Application number: 07795477.4
(22) Date of filing: 30.05.2007
(51) Int. Cl.: A61K 9/16, A61K 9/20, A61K 31/401

(54) **ORAL CONTROLLED RELEASE FORMULATIONS OF AN INTERLEUKIN-1 BETA CONVERTING ENZYME INHIBITOR**
ORALE FORMULIERUNGEN MIT KONTROLLIERTER FREISETZUNG EINES INTERLEUKIN-1-BETA-CONVERTING-ENZYME INHIBITORS
FORMULATIONS À LIBÉRATION CONTRÔLÉE D'UN INHIBITEUR DE L'ENZYME CONVERTANT L' INTERLEUKINE-1-BETA

(30) Priority: 31.05.2006 US 809779 P
(43) Date of publication of application: 25.03.2009
(73) Proprietor: VERTEX PHARMACEUTICALS INCORPORATED, Cambridge, MA 02139-4242 (US)
(72) Inventor: KADIYALA, Irina, Nikolaevna, Newton, MA 02457 (US); LIN, Wu, Wollaton, Nottingham NG8 1HB (GB); HURTER, Patricia, Harvard, MA 01451 (US)
(74) Representative: Cohausz & Florack
(86) International application number: PCT/US2007/012715
(87) International publication number: WO 2007/142951

(56) References cited:
- WO-A-2005/115362
- CORNELIS SIGRID ET AL: "Inflammatory caspases: Targets for novel therapies" CURRENT PHARMACEUTICAL DESIGN, vol. 13, no. 4, 2007, pages 367-385, XP002463834 ISSN: 1381-6128

## Description

### BACKGROUND OF THE INVENTION

The compound of Formula (I), shown below, 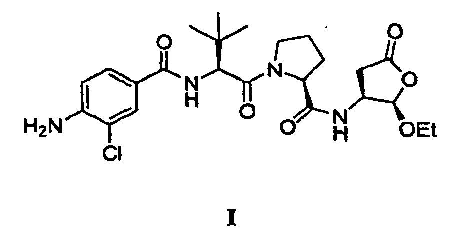 is an oral cytokine inhibitor of interleukin-1 beta converting enzyme, which is targeted at controlling the symptoms and progression of inflammatory diseases, including rheumatoid arthritis and psoriasis. The compound of Formula (I) hydrolyzes *in vivo* to the compound of Formula (IA), shown below.

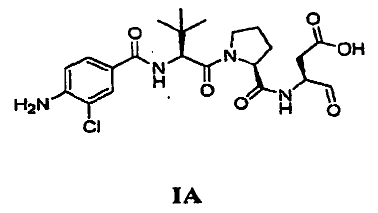

Regional absorption studies of the compound for Formula (I) indicate colonic absorption.

### SUMMARY OF THE INVENTION

In general, the invention relates to a controlled release formulation for an oral cytokine inhibitor of interleukin-1 beta converting enzyme.

In one aspect, the invention includes controlled release formulation and processes for producing the same.

In some embodiments, the process of formulating the compound of Formula (I), 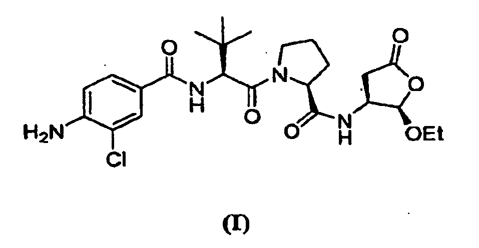 includes granulating the compound of Formula (I) in the presence of an organic solvent; wherein
(a) the stability of the compound of Formula (I) in the organic solvent is such that the solvent does not give rise to degradation of more than 5% (e.g., 2%) of the compound of Formula (I) over 24 hours at or below room temperature,
(b) the wet granulated material has an intrinsic dissolution value of less than about 0.15 mg/min/cm², and
(c) the wet granulated material has a density of between about 0.20 g/cm³ and about 0.90 g/cm³_{.}

Embodiments of these aspects may include one or more of the following features. The density of the wet granulated material can be between about 0.50 g/cm³ and about 0.90 g/cm³. The organic solvent can be isopropyl alcohol. The compound of Formula (I) can be wet granulated with the organic solvent and a first binding agent, wherein the first binding agent can readily be dissolved in both the organic solvent used in wet granulation and in water. The first binding agent can include hydroxypropyl cellulose (e.g., between about 0.5% w/w and about 10% w/w or about 5%) or polyvinylpyrrolidone (e.g., between about 1% and about 20%). The granulation mixture can contain at least 40% (w/w) of the compound of formula I. The granulation mixture can be formulated with a second binding agent, such as polymethacrylate or ethylcellulose, a porous agent, such as lactose or mannitol, a glidant, such as talc, and a lubricant, such as sodium stearyl fumarate. The granulation mixture can be formulated with between about 0% (w/w) to about 20% (w/w) of the second binding agent; between 0% (w/w) and about 20% (w/w) of the porous agent; between about 0.1% (w/w) and about 4% (w/w) of the glidant; and between about 0.1% (w/w) and about 3% (w/w) of the lubricant. The granulation mixture can be formulated with between 5% (w/w) and about 20% (w/w) of the second binding; between about 10% (w/w) and about 20% (w/w) of the porous agent; between about 1% (w/w) and about 2% (w/w) of the glidant; and between about 0.5% (w/w) and about 3% (w/w) of the lubricant. The granulated mixture or formulated granulation mixture can be tableted for formed into pellets. The granulated mixture or formulated granulation mixture can be tableted to a hardness level between about 5 kP and about 16.0 kP.

In some aspects, the formulation can release the compound of Formula (I) for about 8 hours (e.g., ± 2 hours) in a dissolution test such as that described in Example 1. For example, the formulation can release between about 70% and about 90% of the compound of the Formula (I) after about 8 hours in a dissolution test. The formulation can release less than about 40%, e.g., between 5% and 40%, of the compound of Formula (I) with the first 100 minutes in an intrinsic dissolution test. The formulation can release less than about 65%, e.g., between 20% and 60%, of the compound of Formula (I) within the first 300 minutes in a dissolution test. The formulation can release between about 60% to about 80% of the compound of Formula (I) within the first 600 minutes in a dissolution test.

Specific formulations can include about 69.8% (w/w) of the compound of formula I, about 1.6% (w/w) of HPC EXF, about 10% (w/w) of Aqualon T10, about 16.5% (w/w) of Pearlitol 200 SD, about 1.4% (w/w) of Talc, and about 0.7% (w/w) of SSF. The formulated granulation mixture can include about 69.8% (w/w) of the compound of formula I, about 1.6% (w/w) of Klucel EXF, about 15% (w/w) of Eudragit RL PO, about 11.5% (w/w) of Pearlitol 200 SD, about 1.4% (w/w) of Talc, and about 0.7% (w/w) of SSF. Another formulation can include about 94.8% (w/w) of the compound of formula 1, about 2.2% (w/w) of Klucel EXF, about 1.9% (w/w) of Talc, and about 1.1% (w/w) of SSF. Still another formulation can include about 95% of the compound of Formula (I) and about 5% (w/w) of HPC EXF. Yet another formulation can include about 97% of the compound of Formula (I) and about 3% (w/w) of HPC JXF.

Advantageously, the compound of Formula (I) when wet granulated, provides increased density creating improved flow properties.

As used herein, the compound of Formula (I) includes the free form of this compound and its pharmaceutically acceptable salts.

As used herein, the term "organic solvent" includes any solvent except water. Examples of an organic solvent include, without limitation, isopropyl alcohol, hexane, and acetone.

As used herein, the term "% (w/w)" refers to percentage by weight.

As used herein, the term "binding agent" (or interchangeably "retard polymer" or "matrix polymer") refers to agents that can be used as a vehicle for delivery of the compound of Formula (I) in a controlled manner. Suitable agents may be water soluble or insoluble. Examples of the suitable agents include, without limitation, Aqualon T10 Ethylcellulose (Hercules), Klucel hydroxypropylcellulose (HPC), EXF (Hercules), Klucel HPC, JXF (Hercules), Klucel HPC, MXF (Hercules), Klucel HPC, EXF (Hercules), Eudragit RL PO, polymethacrylate (Degussa), and Avicel PH113, microcrystalcellulose (FMC).

As used herein, the term "controlled released" or "sustained release" refers to release of a drug or prodrug from its carrier at a rate or in a manner that is different from the natural rate or carrier, usually slower, and generally preferred to more linear or close to linear. When is released is extended over a long period of time (e.g., 10 hours) in a linear manner (i.e., at a rate that is closely to the same throughout the whole release process), it is also called "sustained release."

Unless otherwise defined, all the terms herein should have the same meanings as those known in the art. All publications cited herein are incorporated in their entirety by reference.

### DETAILED DESCRIPTION OF THE DRAWINGS

Figure 1 illustrates the intrinsic dissolution profiles of three tablet formulations of the invention.

Figure 2 illustrates the intrinsic dissolution profiles of three pellet formulations of the invention.

### DETAILED DESCRIPTION OF THE INVENTION

In general, the controlled release formulations of the present invention include compound of Formula (I) in wet granulated form. Granules of the compound of Formula (I) can be prepared by methods well known in the art. See, e.g., Pharmaceutics: the Science of Dosage Form Design, edited by Michael E. Aulton, Churchill Livingstone (Edinburgh; New York), 2002; and Cyclodextrins in Pharmacy, Karl-Heinz Frömming and József Szejtli, Kluwer Academic Publishers (Dordrecht; Boston), 1994.

In some embodiments, the granules of the compound of Formula (I) are prepared by wet granulation with a non-aqueous solvent, such as an organic solvent. Generally, any organic solvent can be used for wet granulating the compound of Formula (I) provided that the resulting intrinsic dissolution value of a tablet made with the wet granulated material is less than the intrinsic dissolution value of a similar tablet formed with the neat form of the compound of formula I, e.g., the intrinsic dissolution value is less than about 0.15 mg/min/cm², e.g., about 0.095 mg/min/cm². The intrinsic dissolution value can be measured in water at 37 °C according to the U.S. Pharmacopeia (USP) guidelines, e.g., as provided at http://www.usp.org/.

In some embodiments, at least about 50% of the compound of Formula (I) is released from the granules described above within the first 10 hours in an *in vitro* dissolution test as measured by a method known in the art. An example of applicable method is a USP protocol for a delayed release formulations in Apparatus 2 (see, e.g., http://www.usp.org.). An alternative method includes using the paddle method at 50 rpm for up to 10 hours. In still another method, the *in vitro* dissolution values can be determined by using HPLC to assess the dissolved amounts of the compound of Formula (I) and metabolites thereof. The amount of the released compounds can be determined, e.g., by comparing the total peaks area with that of an external control. Examples of columns that can be used in this method include a Waters Symmetry C-18, 50 x 4.6 mm, 3.5 µm column (PN# WAT200625) with mobile phase composed of water, methanol, acetonitrile, and pH 5.0 phosphate buffer, under gradient elution conditions. Detection can be performed by ultraviolet (UV) absorbance at 280 nm. The resulting samples from either method can be analyzed by HPLC. Other methods known in the art can also be used to obtain the release profile of the granules of the compound of Formula (I) or each formulation containing the compound of formula I. See, e.g., Controlled Drug Delivery: Fundamentals and Applications, 2nd Edition, J.R. Robinson and V.H.L. Lee, Marcel Dekker (New York), 1987. For instance, a sample of the formulation can be placed in an aqueous medium and the concentration of the compound of Formula (I) in the medium can be determined, e.g., by measured the activity of interleukin-1 beta converting enzyme, a target enzyme of the compound of formula I.

In other embodiments, any organic solvent can be used to wet granulate the compound of Formula (I) provided that (a) the stability of the compound of Formula (I) in the organic solvent is such that the solvent does not give rise to degradation of more than 5% of the compound of Formula (I) over 24 hours at or below room temperature, (b) the wet granulated material has an intrinsic dissolution value of less than about 0.15 mg/min/cm², and (c) the wet granulated material has a density of between about 0.20 g/cm³ and about 0.90 g/cm³, e.g., between about 0.5 and about 0.65 g/cm³. The increase in density of the granulated material relative to the non-granulated material results in improved flow characteristics which facilitate easier handling during manufacturing processes such as formulation. Bulk and granulated densities can be measured with a measuring cylinder. The values for the bulk (pb) and tap (pt) densities can be used to calculate index of compressibility (% Compressability), which is indicative of the flow properties of the material. The % Compressability can be calculated by the following formula: % Compressability = [(ρt- - pb) / pt-] x 100%. See, e.g., The theory and practice of industrial pharmacy, by L.Lachman, H. Lieberman, and J. Kanig, in Varghese Publishing House, 3rd Indian Edition, (Hind Rajasthan Bldng, Dadar Bombay 400 014), 1987, p.184. Also, flowability of the material can be determined by Flodex analysis. The Flodex Test Apparatus is ideal for determining the intrinsic flowability of powders (which is recommended for quality control of powders). The Flodex instrument includes a complete set of index flow disks with opening of different diameter. A tested powder is allowed to flow through the opening. The ability of the powder to flow through the opening is indicative of the flow properties of the tested powder. See, e.g., Composite Method to Quantify Powder Flow as a Screening Method in Early Tablet or Capsule Formulation Development, by Michael K. Taylor. Jeri Ginsburg, Anthony Hickey, and Ferdous Gheyas, in AAPS PharmSciTech, 2000, 1(3): article 18.

In certain embodiments, the compound of Formula (I) is wet granulated with isopropyl alcohol (IPA).

In conventional wet granulation approaches, most tablet excipients such as fillers, binders and release retard polymers are normally blended with the drug material before granulation. Unlike conventional methods, the material of the compound of Formula (I) is wet granulated prior to full blending with all of the formulation excipients to overcome the low density and poor flowability of the non-granulated material by producing granules with significantly improved density and flowability. The resulting granules are easier to blend with different matrix polymers and other excipients to produce controlled release formulations.

In some embodiments, the compound of Formula (I) is wet granulated with an organic solvent, such as IPA, and the resulting granules are blending with the excipients and other components of the controlled release formulation. In other embodiments, the compound of Formula (I) and first binding agent are wet granulated. Yet in some other embodiments, the compound of Formula (I) can be granulated with the solvent without any binding agents.

The first binding agent can be any agent that dissolves in both the organic solvent used in wet granulation and in water. Examples of the first binding agent include, without limitation, hydroxypropyl cellulose (HPC) and polyvinylpyrrolidone (PVP).

The amount of first binding agent used in the wet granulation can be any amount which provides resulting granules having a size ranging between about 150 µm to about 500 µm, containing greater than about 90 % of the compound of formula I, and a density between 0.35 and about 0.70 g/cm³. In certain embodiments, the compound of Formula (I) is wet granulated with Klucel HPC EXF or PVP (10% in a solution of IPA). In some embodiments, the compound of Formula (I) is wet granulated with between about 0.5% w/w to about 10% w/w, e.g., about 5% w/w, of Klucel HPC EXF or with between about 1% and about 20%, e.g., about 10%, PVP in the solvent, IPA.

Before granulation, the bulk material of the compound of Formula (I) can be sieved, e.g., by using a 500 µm sieve.

In some embodiments, granules of the compound of Formula (I) can be mixed with a binding agent and, optionally, other known pharmaceutical excipients (e.g., other binding agents, a porous agent, and a filler) to obtain a pharmaceutical formulation. When administered in an aqueous medium, such a pharmaceutical formulation is generally capable of releasing the compound of Formula (I) in a controlled manner. Further, depending on the type of the binding agents and other excipients and the weight ratio of the granules of the compound of Formula (I) and the binding agents and other excipients, the pharmaceutical formulation can provide a sustained release of the compound of Formula (I) at a certain level over an extended period of time, e.g., for at least 8 hours or between 8 and 10 hours, such that between about 70% and about 90% of the compound of the Formula (I) is released. The precise release profile of the compound of Formula (I) can be changed by adjusting the type and amount of the binding agents and other excipients contained in the formulation, the hardness of the tablets and pellets. In some embodiments, the controlled release formulations provide release of less than about 40%, e.g., between about 5% to about 40%, of the compound of Formula (I) in the first 100 minutes after placement in a dissolution medium as measured via the dissolution methodology described herein. In certain aspects of these embodiments, the controlled release formulations provide release of less than about 40%, e.g., between about 5% to about 40%, of the compound of Formula (I) in the first 100 minutes; and release of less than about 65%, e.g., between about 20% to about 60%, of the compound of Formula (I) in the first 300 minutes. In still other aspects of these embodiments, the controlled release formulations provide release of less than about 40%, e.g., between about 5% to about 40%, of the compound of Formula (I) in the first 100 minutes; release of less than about 65%, e.g., between about 20% to about 60%, of the compound of Formula (I) in the first 300 minutes; and release of between about 60% to about 80%, of the compound of Formula (I) in the first 600 minutes. In other embodiments, the controlled release formulations provide release of less than about 25%, e.g., between about 5% to about 20%, of the compound of Formula (I) in the first 100 minutes.

Still in some embodiments, the process of formulating granules containing the compound of Formula (I) may be conducted without the presence of an organic or aqueous solvent, i.e., by dry granulation. As a result, the granules thus obtained may contain about 100% (w/w) of the compound of Formula (I), which may also posses the same features or properties as discussed herein

The pharmaceutical formulation can be processed into the form of tablets or pellets for oral administration. The tablets and pellet formulations contain between about 300 mg to about 1000 mg of the compound of formula I.

The sustained release formulations which provide near linear release of the compound of Formula (I) over about 10 hours includes pellets or tables of granules resultant from wet granulation of the compound of Formula (I) with less than about 3% w/w hydroxypropyl cellulose. In some embodiments, the tablets containing the sustained release formulations have a hardness less than about 10 kP, e.g., the tables have a hardness between about 6.5 and about 9.0 kP. In certain embodiments, the tablet hardness is between about 7.0 kP and about 9.0 kP.

The formulation may also include additional excipients including, without limitations, additional binding agents, e.g., polymethacrylate or ethylcellulose; porous agents, e.g., lactose or mannitol; glidants, e.g., talc; and lubricants, e.g., sodium stearyl fumarate (SSF).

In some embodiments, the formulation containing wet granulated compound of Formula (I) includes a second binding agent in an amount between about 0% (w/w) to about 20% (w/w), e.g., between 5% (w/w) to about 20% (w/w); a porous agent in an amount between 0% (w/w) to about 20% (w/w), e.g., between about 10% (w/w) to about 20% (w/w); a glidant in an amount between about 0.1 % (w/w) to about 4% (w/w), e.g., between about 1% (w/w) to about 2% (w/w); and a lubricant in an amount between about 0.1% (w/w) to about 3% (w/w), e.g., between about 0.5% (w/w) to about 1.5% (w/w).

Not intended to limit the scope of the claimed invention, examples of the invention are set forth below for illustration purpose.

### Example 1: Dissolution Values

The dissolution test method was derived from the USP dissolution monograph for modified release dosage forms. Apparatus 2 (paddle method) was employed at 50 rpm for up to 10 hours. Analysis of the resultant samples was performed by HPLC.

The HPLC method assessed the amount of the compound of Formula (I) and metabolites thereof present in dissolution samples, using a Waters Symmetry C-18, 50 x 4.6 mm, 3.5 □ m column (Part No. WAT200625) with mobile phase composed of water, methanol, acetonitrile, and phosphate buffer pH 5.0, under gradient elution conditions. At a flow of 2.5 mL/minute and a column temperature of 45 °C, the retention time of the compound of Formula (I) and metabolites thereof was approximately 3.7 minutes and 1.5 minutes respectively. Detection was performed by ultraviolet (UV) absorbance at 280 nm. The release of the compound of Formula (I) was calculated by comparison of the total peak area of the compound of Formula (I) and metabolites thereof and with an external standard solution of the compound of formula I.

### Example 2: Bulk Non-Granulated Materials

The bulk material of the compound of Formula (I) was found as white powder with some aggregates. The material was first sieved through a 500 µm sieve. The density of the 500 µm sieved bulk material was found to be around 0.24 g/cm³ with very poor flowability. The morphology of the material was investigated under an optical microscope and it was found that majority of the bulk material are aggregates from much smaller crystals. These aggregates are highly porous and hence generate a low apparent density and poor flowability, which makes the direct compression for tableting very difficult.

### Example 3: Wet Granulation of the Compound of Formula (I)

To prepare the Compound of Formula (I) granules by wet granulation, bulk material of the compound of Formula (I) (500 µm sieved) can be placed in a mortar and a first binding agent (e.g., HPC EXF at 5% w/w or PVP at 10% w/w) solution in isopropyl alcohol (IPA) is then added dropwise and mixed using a pestle until the end point of granulation is reached. The mass is then manually passed through a sieve (e.g., 1.18 mm) and dried at room temperature overnight. The granules are then passed through a finer sieve (e.g., 500 µm) to break any aggregates. The large particles can be milled by using a mortar and pestle, and again sieved through the finer sieve. The granules thus prepared are then sieved again to remove the fraction of powder that is, e.g., smaller than 150 µm. Bulk and granule densities and flowability of the final granules can be examined as described above. The granules of the Compound of Formula (I) are stored in an amber bottle at room temperature.

Compared to the bulk form of the Compound of formula I, granules produced from wet granulation have higher density and better flowability and thus are more suitable for high-dosage administration. For instance, depending on the amount of the binding agent used in granulation and the length of the granulation, the density of granules of the Compound of Formula (I) can be increased to, e.g., at least 0.4 g/cm³ (e.g., 0.5-0.65 g/cm³), from about 0.2 g/cm³ of the natural form.

### Example 4: Preparation of tablets for controlled release of the Compound of Formula (I)

The following tablets were prepared using the wet granulated material and the excipients:

| **Tablet** | **Composition / tablet** | **Tablet size** | **Hardness** |
|---|---|---|---|
| 991-26-1 | Wet Granulation: the compound of Formula (I) (600 mg, | 18 x 9 mm 860 mg | 7.6 KP |
| | 69.8%), and HPC EXF (1.6%) with IPA, | | |
| | Aqualon T10 (10.0%), | | |
| | Pearlitol 200 SD (16.5%), | | |
| | Talc (1.4%), | | |
| | SSF (0.7%) | | |
| 991-26-5 | Wet Granulation: the compound of Formula (I) (600 mg, | 18 x 9 mm 860 mg | 8.6KP |
| | 69.8%) and Klucel EXF (1.6%) with IPA, | | |
| | Eudragit RL PO (15.0%), | | |
| | Pearlitol 200 SD (11.5%), | | |
| | Talc (1.4%), | | |
| | SSF (0.7%) | | |
| 991-26-6 | Wet Granulation: the compound of Formula (I) (600 mg, | 18 x 9 mm 633 mg | 7.8 KP |
| | 94.8%) and Klucel EXF (2.2%) with IPA, | | |
| | (1.9%) Talc (1.9%), | | |
| | SSF (1.1%) | | |

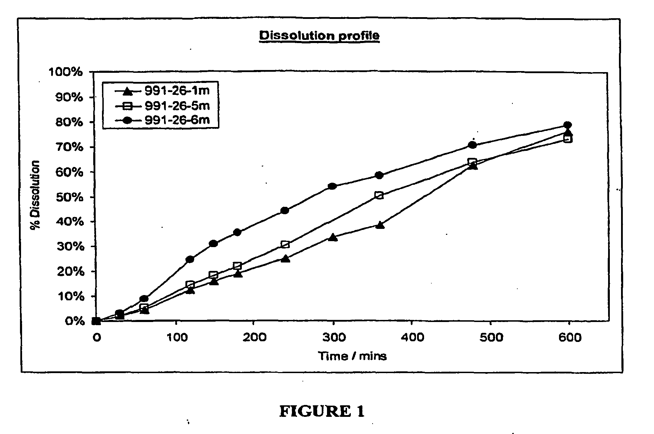

Figure 1 illustrates the dissolution profiles for 10 hours. The three tablets exhibited good dissolution profiles, i.e., nearly linear, releasing between about 70% to about 80% of the compound of Formula (I) over the 10-hour period.

### Example 5: Preparation of pellet formulations

Pellets were prepared at a 30-gram scale. The size fraction of the pellet formulation was 800/1180 µm. The details of the pellet formulations are included in the following table. The density of the final pellets was examined and the pellets were stored in a vial before the dissolution test.

The following pellets were prepared using the wet granulated material as indicated below:

| **Formulation** | **Ingredients** | **Density of pellets (g/cm³)** |
|---|---|---|
| 991-29-2 | Wet granulation of the compound of Formula (I) with HPC EXF (95:5) in IPA | 0.51 |
| 991-29-3 | Wet granulation of the compound of Formula (I) with HPC JXF (97:3) in IPA | 0.62 |
| 991-31-1 | Wet granulation of the compound of Formula (I) with IPA | 0.61 |

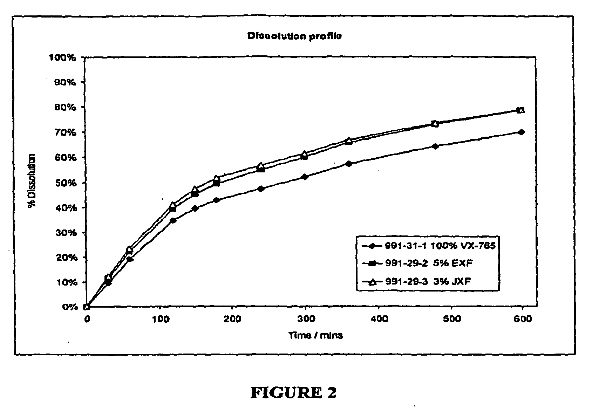

Figure 2 illustrates the dissolution profiles of Formulations 991-29-2, 991-29-3, and 991-31-1 for 10 hours. The three pellet formulations exhibited good dissolution profiles, i.e., quasi-linear, releasing between about 60% to about 80% of the compound of Formula (I) over the 10 hour period.

## Claims

1. A process of formulating the compound of Formula (I), 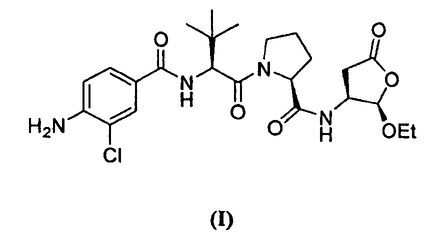 comprising:
granulating the compound of Formula (I) in the presence of an organic solvent; wherein
(a) the stability of the compound of Formula (I) in the organic solvent is such that the solvent does not give rise to degradation of more than 5% of the compound of Formula (I) over 24 hours at or below room temperature,
(b) the wet granulated material has an intrinsic dissolution value of less than about 0.15 mg/min/cm², and
(c) the wet granulated material has a density of between about 0.20 g/cm³ and about 0.90 g/cm³.

2. The process of claim 1, wherein the organic solvent is isopropyl alcohol.

3. The process of claim 1, wherein the compound of Formula (I) is wet granulated with the organic solvent and a first binding agent, wherein the first binding agent dissolves both in the organic solvent used in wet granulation and in water and comprises hydroxypropyl cellulose or polyvinylpyrrolidone.

4. The process of claim 1, wherein the formulated granulation mixture contains at least 40% (w/w) of the compound of Formula (1) by weight

5. The process of claim 1, further comprising formulating the granulation mixture with a second binding agent, wherein the second binding agent comprises polymethacrylate or ethylcellulose.

6. The process of claim 1, further comprising formulating the granulation mixture with a porous agent, wherein the porous agent comprises lactose or mannitol.

7. The process of claim 1, further comprising formulating the granulation mixture with a glidant, wherein the glidant comprises talc.

8. The process of claim 1, further comprising formulating the granulation mixture with a lubricant, wherein the lubricant comprises sodium stearyl fumarate.

9. The process of claim 1, further comprising formulating the granulation mixture with a second binding agent, a porous agent, a glidant, and a lubricant, wherein the second binding agent comprises polymethacrylate or ethylcellulose, the porous agent comprises lactose or mannitol, the glidant comprises talc, and the lubricant comprises sodium stearyl fumarate.

10. The process of claim 9, wherein the granulation mixture is formulated with between 5% (w/w) to about 20% (w/w) of the second binding; between about 10% (w/w) to about 20% (w/w) of the porous agent; between about 1% (w/w) to about 2% (w/w) of the glidant; and between about 0.5% (w/w) to about 1.5% (w/w) of the lubricant.

11. The process of claim 1, wherein the formulated granulation mixture comprises about 69.8% of the compound of Formula (I), about 1.6 % of HPC EXF, about 10% Aqualon T10, about 16.5% Pearlitol 200 SD, about 1.4% Talc, and about 0.7% SSF.

12. The process of claim 1, wherein the formulated granulation mixture comprises about 69.8% of the compound of formula I, about 1.6% Klucel EXF, about 15% Eudragit RL PO, about 11.5% Pearlitol 200 SD, about 1.4% Talc, and about 0.7% SSF.

13. The process of claim 1, wherein the formulated granulation mixture comprises about 94.8% of the compound of formula I, about 2.2% Klucel EXF, about 1.9% Talc, and about 1.1% SSF.

14. The process of claim 1, wherein the formulated granulation mixture comprises about 95% of the compound of Formula (I) and about 5% of HPC EXF.

15. The process of claim 1, wherein the formulated granulation mixture comprises about 97% of the compound of Formula (I) and about 3% of HPC JXF.

16. The process of claim 1, wherein the granulated mixture or formulated granulation mixture is tableted to a hardness between about 6.5 kP to about 16.0 kP.

17. The process of claim 1, further comprising forming pellets with the granulated mixture or formulated granulation mixture.

18. The process of claim 16 or 17, wherein the formulation releases the compound of Formula (I) after about 8 hours in a dissolution test.

19. The process of claim 16 or 17, wherein the formulation releases less than about 40% of the compound of Formula (I) within the first 100 minutes in a dissolution test.

20. The process of claim 16 or 17, wherein the formulation releases less than about 65% of the compound of Formula (I) within the first 300 minutes in a dissolution test.

21. The process of claim 16 or 17, wherein the formulation releases between about 60% to about 80% of the compound of Formula (I) within the first 600 minutes in a dissolution test.

22. The process of claim 1, wherein at least 50% of the compound of Formula (I) is released from the granulated mixture or formulated granulation mixture after the first 10 hours in a dissolution test.

23. A controlled release formulation, comprising a wet granulated material which comprises the compound of Formula (I), 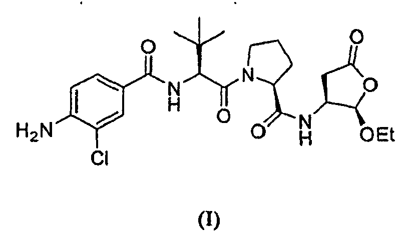 wherein the wet granulated material exhibits an intrinsic dissolution value of less than about 0.15 mg/min/cm², and a density between about 0.20 g/cm³ and about 0.90 g/cm³.

24. The controlled release formulation of claim 23, wherein the wet granulated material further comprises a first binding agent, wherein the first binding agent dissolves in both the organic solvent used in wet granulation and in water, and wherein the first binding agent comprises hydroxypropyl cellulose or polyvinylpyrrolidone.

25. The controlled release formulation of claim 23, wherein the wet granulated material contains at least 85% (w/w) of the compound of formula I.

26. The controlled release formulation of claim 23, further comprising a second binding agent, wherein the second binding agent comprises polymethacrylate or ethylcellulose.

27. The controlled release formulation of claim 26, further comprising a porous agent, wherein the porous agent comprises lactose or mannitol.

28. The controlled release formulation of claim 26, further comprising a glidant, wherein the glidant comprises talc.

29. The controlled release formulation of claim 26, further comprising a lubricant, wherein the lubricant comprises sodium stearyl fumarate.

30. The controlled release formulation of claim 23, further comprising a second binding agent, a porous agent, a glidant, and a lubricant, wherein the formulation comprises between about 0% (w/w) and about 20% (w/w) of the second binding agent; between 0% (w/w) and about 20% (w/w) of the porous agent; between about 0.1% (w/w) and about 4% (w/w) of the glidant; and between about 0.1% (w/w) and about 3% (w/w) of the lubricant.

31. A controlled release formulation, comprising about 69.8% (w/w) of the compound of Formula (I), about 1.6 % (w/w) of HPC EXF, about 10% (w/w) of Aqualon T10, about 16.5% (w/w) of Pearlitol 200 SD, about 1.4% (w/w) of Talc, and about 0.7% (w/w) of SSF, wherein the compound of Formula (I) and HPC EXF are wet granulated with isopropyl alcohol.

32. A controlled release formulation, comprising about 69.8% (w/w) of the compound of Formula (I), about 1.6% (w/w) of Klucel EXF, about 15% (w/w) of Eudragit RL PO, about 11.5% (w/w) of Pearlitol 200 SD, about 1.4% (w/w) of Talc, and about 0.7% (w/w) of SSF, wherein the compound of Formula (I) and Klucel EXF are wet granulated with isopropyl alcohol.

33. A controlled release formulation, comprising about 94.8% (w/w) of the compound of Formula (I), about 2.2% (w/w) of Klucel EXF, about 1.9% (w/w) of Talc, and about 1.1% (w/w) of SSF, wherein the compound of Formula (I) and Klucel EXF are wet granulated with isopropyl alcohol

34. The controlled release formulation of claim 31, 32, or 33 wherein the mixture is tableted to a hardness of less than about 16 kP.

35. A controlled release formulation, comprising pellets including wet granulated material, wherein the wet granulated material comprises 95% of the compound of Formula (I) and about 5% of HPC EXF granulated in isopropyl alcohol.

36. A controlled release formulations, comprising pellets including wet granulated material, wherein the wet granulated material comprises about 97% of the compound of Formula (I) and about 3% of HPC JXF granulated in isopropyl alcohol.

37. The controlled release formulation of claim 23, 31, 32, or 33, wherein the formulation releases between about 70% and about 90% of the compound of the Formula (I) after about 8 hours in dissolution test.

38. The controlled release formulation of claim 23, 31, 32, or 33, wherein the formulation releases less than about 40% of the compound of Formula (I) within the first 100 minutes in a dissolution test.

39. The controlled release formulation of claim 23, 31, 32, or 33, wherein the formulation releases less than about 65% of the compound of Formula (I) within the first 300 minutes in a dissolution test.

40. The controlled release formulation of claim 23, 31, 32, or 33, wherein at least 50% of the compound of Formula (I) is released from the formulation within the first 10 hours in a dissolution test.

## Patentansprüche

1. Verfahren zur Formulierung der Verbindung mit der Formel (I) 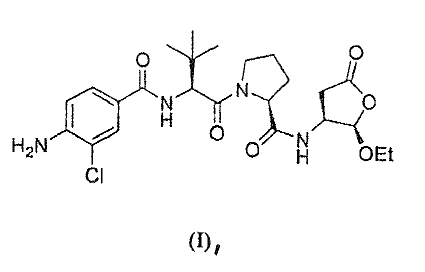 welches das Granulieren der Verbindung mit der Formel (I) in Gegenwart eines organischen Lösungsmittels umfasst, wobei:
a) die Stabilität der Verbindung mit der Formel (I) in dem organischen Lösungsmittel derart ist, dass das Lösungsmittel über einen Zeitraum von 24 Stunden bei oder unterhalb der Raumtemperatur keinen Abbau von mehr als 5 % der Verbindung mit der Formel (I) verursacht, und
b) der innere Auflösungswert des nass granulierten Materials weniger als etwa 0,15 mg/min/cm² und
c) die Dichte des nass granulierten Materials zwischen etwa 0,20 g/cm³ und etwa 0,90 g/cm³ beträgt.

2. Verfahren nach Anspruch 1, wobei das organische Lösungsmittel Isopropylalkohol ist.

3. Verfahren nach Anspruch 1, wobei die Verbindung mit der Formel (I) mit dem organischen Lösungsmittel und einem ersten Bindemittel nass granuliert wird, wobei sich das erste Bindemittel sowohl in dem beim Nassgranulieren verwendeten organischen Lösungsmittel als auch in Wasser löst und Hydroxypropylcellulose oder Polyvinylpyrrolidon enthält.

4. Verfahren nach Anspruch 1, wobei das formulierte Granulatgemisch gewichtsmäßig mindestens 40 % (Gew./Gew.) Verbindung mit der Formel (I) enthält.

5. Verfahren nach Anspruch 1, das weiterhin die Formulierung des Granulatgemischs mit einem zweiten Bindemittel umfasst, das Polymethacrylat oder Ethylcellulose enthält.

6. Verfahren nach Anspruch 1, das ferner die Formulierung des Granulatgemischs mit einem porösen Mittel umfasst, das Lactose oder Mannit enthält.

7. Verfahren nach Anspruch 1, das außerdem die Formulierung des Granulatgemischs mit einem Gleitmittel umfasst, das Talk enthält.

8. Verfahren nach Anspruch 1, das zusätzlich die Formulierung des Granulatgemischs mit einem Schmiermittel umfasst, das Natriumstearylfumarat enthält.

9. Verfahren nach Anspruch 1, das weiterhin die Formulierung des Granulatgemischs mit einem zweiten Bindemittel, einem porösen Mittel, einem Gleitmittel und einem Schmiermittel umfasst, wobei das zweite Bindemittel Polymethacrylat oder Ethylcellulose, das poröse Mittel Lactose oder Mannit, das Gleitmittel Talk und das Schmiermittel Natriumstearylfumarat enthält.

10. Verfahren nach Anspruch 9, wobei das Granulatgemisch mit zwischen 5 % (Gew./Gew.) und etwa 20 % (Gew./Gew.) des zweiten Bindemittels, zwischen etwa 10 % (Gew./Gew.) und etwa 20 % (Gew./Gew.) des porösen Mittels, zwischen etwa 1 % (Gew./Gew.) und etwa 2 % (Gew./Gew.) des Gleitmittels und zwischen etwa 0,5 % (Gew./Gew.) und etwa 1,5 % (Gew./Gew.) des Schmiermittels formuliert wird.

11. Verfahren nach Anspruch 1, wobei das formulierte Granulatgemisch etwa 69,8 % der Verbindung mit der Formel (I), etwa 1,6 % HPC EXF, etwa 10 % Aqualon T10, etwa 16,5 % Pearlitol 200 SD, etwa 1,4 % Talk und etwa 0,7 % SSF enthält.

12. Verfahren nach Anspruch 1, wobei das formulierte Granulatgemisch etwa 69,8 % der Verbindung mit der Formel (I), etwa 1,6 % Klucel EXF, etwa 15 % Eudragit RL PO, etwa 11,5 % Pearlitol 200 SD, etwa 1,4 % Talk und etwa 0,7 % SSF enthält.

13. Verfahren nach Anspruch 1, wobei das formulierte Granulatgemisch etwa 94,8 % der Verbindung mit der Formel (I), etwa 2,2 % Klucel EXF, etwa 1,9 % Talk und etwa 1,1 % SSF enthält.

14. Verfahren nach Anspruch 1, wobei das formulierte Granulatgemisch etwa 95 % der Verbindung mit der Formel (I) und etwa 5 % HPC EXF enthält.

15. Verfahren nach Anspruch 1, wobei das formulierte Granulatgemisch etwa 97 % der Verbindung mit der Formel (I) und etwa 3 % HPC JXF enthält.

16. Verfahren nach Anspruch 1, wobei das Granulatgemisch oder formulierte Granulatgemisch auf eine Härte von zwischen etwa 6,5 kP und etwa 16,0 kP tablettiert wird.

17. Verfahren nach Anspruch 1, das weiterhin das Formen von Pellets aus dem Granulatgemisch oder formulierten Granulatgemisch umfasst.

18. Verfahren nach Anspruch 16 oder 17, wobei in einem Auflösungsversuch die Verbindung mit der Formel (I) nach etwa 8 Stunden von der Formulierung freigesetzt wird.

19. Verfahren nach Anspruch 16 oder 17, wobei in einem Auflösungsversuch weniger als etwa 40 % der Verbindung mit der Formel (I) innerhalb der ersten 100 Minuten von der Formulierung freigesetzt werden.

20. Verfahren nach Anspruch 16 oder 17, wobei in einem Auflösungsversuch weniger als etwa 65 % der Verbindung mit der Formel (I) innerhalb der ersten 300 Minuten von der Formulierung freigesetzt werden.

21. Verfahren nach Anspruch 16 oder 17, wobei in einem Auflösungsversuch zwischen etwa 60 % und etwa 80 % der Verbindung mit der Formel (I) innerhalb der ersten 600 Minuten von der Formulierung freigesetzt werden.

22. Verbindung nach Anspruch 1, wobei in einem Auflösungsversuch mindestens 50 % der Verbindung mit der Formel (I) nach den ersten 10 Stunden von dem Granulatgemisch oder formulierten Granulatgemisch freigesetzt werden.

23. Formulierung mit kontrollierter Freisetzung, die ein nass granuliertes Material enthält, das die Verbindung mit der Formel (I) 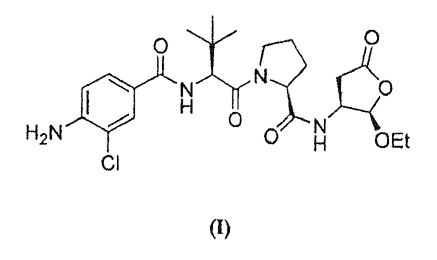 enthält, wobei das nass granulierte Material einen inneren Auflösungswert von weniger als etwa 0,15 mg/min/cm² und eine Dichte von zwischen etwa 0,20 g/cm³ und etwa 0,90 g/cm³ besitzt.

24. Formulierung mit kontrollierter Freisetzung nach Anspruch 23, wobei das nass granulierte Material ferner ein erstes Bindemittel enthält, das sich sowohl in dem beim Nassgranulieren verwendeten organischen Lösungsmittel als auch in Wasser löst und Hydroxypropylcellulose oder Polyvinylpyrrolidon enthält.

25. Formulierung mit kontrollierter Freisetzung nach Anspruch 23, wobei das nass granulierte Material mindestens 85 % (Gew./Gew.) der Verbindung mit der Formel (I) enthält.

26. Formulierung mit kontrollierter Freisetzung nach Anspruch 23, die weiterhin ein zweites Bindemittel enthält, das Polymethacrylat oder Ethylcellulose enthält.

27. Formulierung mit kontrollierter Freisetzung nach Anspruch 26, die ferner ein poröses Mittel enthält, das Lactose oder Mannit enthält.

28. Formulierung mit kontrollierter Freisetzung nach Anspruch 26, die außerdem ein Gleitmittel enthält, das Talk enthält.

29. Formulierung mit kontrollierter Freisetzung nach Anspruch 26, die zusätzlich ein Schmiermittel enthält, das Natriumstearylfumarat enthält.

30. Formulierung mit kontrollierter Freisetzung nach Anspruch 23, die weiterhin ein zweites Bindemittel, ein poröses Mittel, ein Gleitmittel und ein Schmiermittel enthält, wobei die Formulierung zwischen etwa 0 % (Gew./Gew.) und etwa 20 % (Gew./Gew.) des zweiten Bindemittels, zwischen 0 % (Gew./Gew.) und etwa 20 % (Gew./Gew.) des porösen Mittels, zwischen etwa 0,1 % (Gew./Gew.) und etwa 4 % (Gew./Gew.) des Gleitmittels und zwischen etwa 0,1 % (Gew./Gew.) und etwa 3 % (Gew./Gew.) des Schmiermittels enthält.

31. Formulierung mit kontrollierter Freisetzung, die etwa 69,8 % (Gew./Gew.) der Verbindung mit der Formel (I), etwa 1,6 % (Gew./Gew.) HPC EXF, etwa 10 % (Gew./Gew.) Aqualon T10, etwa 16,5 % (Gew./Gew.) Pearlitol 200 SD, etwa 1,4 % (Gew./Gew.) Talk und etwa 0,7 % (Gew./Gew.) SSF enthält, wobei die Verbindung mit der Formel (I) und HPC EXF mit Isopropylalkohol nass granuliert worden sind.

32. Formulierung mit kontrollierter Freisetzung, die etwa 69,8 % (Gew./Gew.) der Verbindung mit der Formel (I), etwa 1,6 % (Gew./Gew.) Klucel EXF, etwa 15 % (Gew./Gew.) Eudragit RL PO, etwa 11,5 % (Gew./Gew.) Pearlitol 200 SD, etwa 1,4 % (Gew./Gew.) Talk und etwa 0,7 % (Gew./Gew.) SSF enthält, wobei die Verbindung mit der Formel (I) und Klucel EXF mit Isopropylalkohol nass granuliert worden sind.

33. Formulierung mit kontrollierter Freisetzung, die etwa 94,8 % (Gew./Gew.) der Verbindung mit der Formel (I), etwa 2,2 % (Gew./Gew.) Klucel EXF, etwa 1,9 % (Gew./Gew.) Talk und etwa 1,1 % (Gew./Gew.) SSF enthält, wobei die Verbindung mit der Formel (I) und Klucel EXF mit Isopropylalkohol nass granuliert worden sind.

34. Formulierung mit kontrollierter Freisetzung nach Anspruch 31, 32 oder 33 wobei das Gemisch auf eine Härte von weniger als etwa 16 kP tablettiert worden ist.

35. Formulierung mit kontrollierter Freisetzung, die nass granuliertes Material enthaltende Pellets enthält, wobei das nass granulierte Material 95 % der Verbindung mit der Formel (I) und etwa 5 % HPC EXF, granuliert in Isopropylalkohol, enthält.

36. Formulierung mit kontrollierter Freisetzung, die nass granuliertes Material enthaltende Pellets enthält, wobei das nass granulierte Material etwa 97 % der Verbindung mit der Formel (I) und etwa 3 % HPC JXF, granuliert in Isopropylalkohol, enthält.

37. Formulierung mit kontrollierter Freisetzung nach Anspruch 23, 31, 32 oder 33, wobei im Auflösungsversuch nach etwa 8 Stunden zwischen etwa 70 % und etwa 90 % der Verbindung mit der Formel (I) von der Formulierung freigesetzt werden.

38. Formulierung mit kontrollierter Freisetzung nach Anspruch 23, 31, 32 oder 33, wobei in einem Auflösungsversuch innerhalb der ersten 100 Minuten weniger als etwa 40 % der Verbindung mit der Formel (I) von der Formulierung freigesetzt werden.

39. Formulierung mit kontrollierter Freisetzung nach Anspruch 23, 31, 32 oder 33, wobei in einem Auflösungsversuch innerhalb der ersten 300 Minuten weniger als etwa 65 % der Verbindung mit der Formel (I) von der Formulierung freigesetzt werden.

40. Formulierung mit kontrollierter Freisetzung nach Anspruch 23, 31, 32 oder 33, wobei in einem Auflösungsversuch innerhalb der ersten 10 Stunden mindestens 50 % der Verbindung mit der Formel (I) von der Formulierung freigesetzt werden.

## Revendications

1. Procédé de formation d'une préparation du composé de
formule (I) 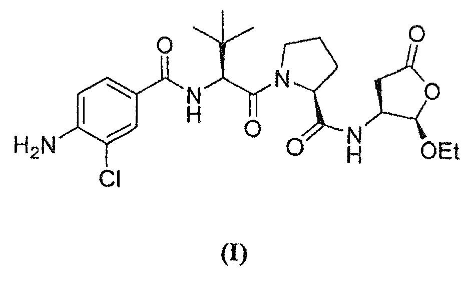 comprenant
la granulation du composé de formule (I) en présence d'un solvant organique, tandis que
(a) la stabilité du composé de formule (I) dans le solvant organique est telle que le solvant ne dégrade pas plus de 5% du composé de formule (I) en 24 heures, à ou en dessous de la température ambiante,
(b) la matière granulée par voie humide présente une valeur de dissolution intrinsèque inférieure à environ 0,15 mg/min/cm³, et
(c) la matière granulée par voie humide présente une masse spécifique entre environ 0,20 g/cm³ et environ 0,90 g/cm³.

2. Procédé selon la revendication 1, dans lequel le solvant organique est l'alcool isopropylique.

3. Procédé selon la revendication 1, dans lequel le composé de formule (I) est granulé par voie humide avec le solvant organique et un premier agent liant, le premier agent liant se dissolvant à la fois dans le solvant organique utilisé dans la granulation par voie humide, et dans l'eau, et comprenant de l'hydroxypropyl cellulose ou du polyvinylpyrrolidone.

4. Procédé selon la revendication 1, dans lequel le mélange de granulation préparé contient au moins 40% (masse/masse) du composé de formule (I), en masse.

5. Procédé selon la revendication 1, comprenant de plus la formation du mélange de granulation avec un second agent liant, le second agent liant comprenant du polyméthacrylate ou de l'éthylcellulose.

6. Procédé selon la revendication 1, comprenant de plus la formation du mélange de granulation avec un agent de porosité, l'agent de porosité comprenant du lactose ou du mannitol.

7. Procédé selon la revendication 1, comprenant de plus la formation du mélange de granulation avec un agent glissant, l'agent glissant comprenant du talc.

8. Procédé selon la revendication 1, comprenant de plus la formation du mélange de granulation avec un lubrifiant, le lubrifiant comprenant du stéaryl fumarate de sodium.

9. Procédé selon la revendication 1, comprenant de plus la formation du mélange de granulation avec un second agent liant, un agent de porosité, un agent glissant et un lubrifiant, le second agent liant comprenant du polyméthacrylate ou de l'éthylcellulose, l'agent de porosité comprenant du lactose ou du mannitol, l'agent glissant comprenant du talc, et le lubrifiant comprenant du stéaryl fumarate de sodium.

10. Procédé selon la revendication 9, dans lequel le mélange de granulation est préparé avec entre 5% (masse/masse) et environ 20% (masse/masse) du second agent liant; entre environ 10% (masse/masse) et environ 20% (masse/masse) de l'agent de porosité ; entre environ 1% (masse/masse) et environ 2% (masse/masse) de l'agent glissant ; et entre environ 0,5% (masse/masse) et environ 1,5% (masse/masse) du lubrifiant.

11. Procédé selon la revendication 1, dans lequel le mélange de granulation préparé comprend environ 69,8% du composé de formule (I), environ 1,6% HPC EXF, environ 10% Aqualon T10, environ 16,5% Pearlitol 200 SD, environ 1,4% talc et environ 0,7% SSF.

12. Procédé selon la revendication 1, dans lequel le mélange de granulation préparé comprend environ 69,8% du composé de formule (I), environ 1,6% Klucel EXF, environ 15% Eudragit RL PO, environ 11,5% Pearlitol 200 SD, environ 1,4% Talc, et environ 0,7% SSF.

13. Procédé selon la revendication 1, dans lequel le mélange de granulation préparé comprend environ 94,8% du composé de formule (I), environ 2,2% Klucel EXF, environ 1,9% talc, et environ 1,1% SSF.

14. Procédé selon la revendication 1, dans lequel le mélange de granulation préparé comprend environ 95% du composé de formule (I) et environ 5% HPC EXF.

15. Procédé selon la revendication 1, dans lequel le mélange de granulation préparé comprend environ 97% du composé de formule (I) et environ 3% HPC JXF.

16. Procédé selon la revendication 1, dans lequel le mélange granulé ou le mélange de granulation préparé est transformé en pastilles jusqu'à une dureté entre environ 6,5 kP et environ 16,0 kP.

17. Procédé selon la revendication 1, comprenant de plus la formation de pellets avec le mélange granulé ou le mélange de granulation préparé.

18. Procédé selon la revendication 16 ou 17, dans lequel la préparation libère le composé de formule (I) après environ 8 heures dans un test de dissolution.

19. Procédé selon la revendication 16 ou 17, dans lequel la préparation libère moins d'environ 40% du composé de formule (I) au cours des 100 premières minutes dans un test de dissolution.

20. Procédé selon la revendication 16 ou 17, dans lequel la préparation libère moins d'environ 65% du composé de formule (I) au cours des 300 premières minutés dans un test de dissolution.

21. Procédé selon la revendication 16 ou 17, dans lequel la préparation libère moins entre environ 60% et environ 80% du composé de formule (I) au cours des 600 premières minutés dans un test de dissolution.

22. Procédé selon la revendication 1, dans lequel au moins 50% du composé de formule (I) sont libérés du mélange granulé ou du mélange de granulation préparé après les 10 premières heures dans un test de dissolution.

23. Préparation à libération contrôlée, comprenant une matière granulée par voie humide qui comprend le composé de formule (I) 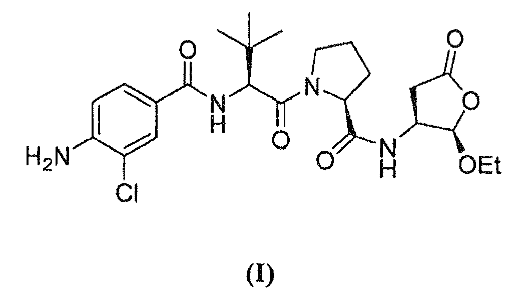 dans laquelle la matière granulée par voie humide présente une valeur de dissolution intrinsèque inférieure à environ 0,15 mg/min/cm³, et une masse spécifique entre environ 0,20 g/cm³ et environ 0,90 g/cm³.

24. Préparation à libération contrôlée selon la revendication 23, dans laquelle la matière granulée par voie humide comprend de plus un premier agent liant, le premier agent liant se dissolvant à la fois dans le solvant organique utilisé dans la granulation par voie humide, et dans l'eau, et le premier agent liant comprenant de l'hydroxypropyl cellulose ou du polyvinylpyrrolidone.

25. Préparation à libération contrôlée selon la revendication 23, dans laquelle la matière granulée par voie humide contient au moins 85% (masse/mass) du composé de formule (I).

26. Préparation à libération contrôlée selon la revendication 23, comprenant de plus un second agent liant, le second agent liant comprenant du polyméthacrylate ou de l'éthylcellulose.

27. Préparation à libération contrôlée selon la revendication 26, comprenant de plus un agent de porosité, l'agent de porosité comprenant du lactose ou du mannitol.

28. Préparation à libération contrôlée selon la revendication 26, comprenant de plus un agent glissant, l'agent glissant comprenant du talc.

29. Préparation à libération contrôlée selon la revendication 26, comprenant de plus un lubrifiant, le lubrifiant comprenant du stéaryl fumarate de sodium.

30. Préparation à libération contrôlée selon la revendication 23, comprenant de plus un second agent liant, un agent de porosité, un agent glissant et un lubrifiant, la préparation comprenant entre environ 0% (masse/masse) et environ 20% (masse/masse) du second agent liant; entre environ 0% (masse/masse) et environ 20% (masse/masse) de l'agent de porosité ; entre environ 0,1% (masse/masse) et environ 4% (masse/masse) de l'agent glissant ; et entre environ 0,1% (masse/masse) et environ 3% (masse/masse) du lubrifiant.

31. Préparation à libération contrôlée, comprenant environ 69,8% (masse/masse) du composé de formule (I), environ 1,6% (masse/masse) HPC EXF, environ 10% (masse/masse) Aqualon T10, environ 16,5% (masse/masse) Pearlitol 200 SD, environ 1,4% (masse/masse) talc et environ 0,7% (masse/masse) SSF, le composé de formule (I) et HPC EXF étant granulés par voie humide avec de l'alcool isopropylique.

32. Préparation à libération contrôlée, comprenant environ 69,8% (masse/masse) du composé de formule (I), environ 1,6% (masse/masse) Klucel EXF, environ 15% (masse/masse) Eudragit RL PO, environ 11,5% (masse/masse) Pearlitol 200 SD, environ 1,4% (masse/masse) talc, et environ 0,7% (masse/masse) SSF, le composé de formule (I) et Klucel EXF étant granulés par voie humide avec de l'alcool isopropylique.

33. Préparation à libération contrôlée, comprenant environ 94,8% (masse/masse) du composé de formule (I), environ 2,2% (masse/masse) Klucel EXF, environ 1,9% (masse/masse) talc, et environ 1,1% (masse/masse) SSF, le composé de formule (I) et Klucel EXF étant granulés par voie humide avec de l'alcool isopropylique.

34. Préparation à libération contrôlée selon la revendication 31, 32 ou 33, dans laquelle le mélange est transformé en pellets d'une dureté inférieure à environ 16 kP.

35. Préparation à libération contrôlée comprenant des pellets incluant une matière granulée par voie humide, dans laquelle la matière granulée par voie humide contient 95% du composé de formule (I) et environ 5% HPC EXF granulé dans l'alcool isopropylique.

36. Préparation à libération contrôlée comprenant des pellets incluant une matière granulée par voie humide, dans laquelle la matière granulée par voie humide contient environ 97% du composé de formule (I) et environ 3% HPC JXF granulé dans l'alcool isopropylique.

37. Préparation à libération contrôlée selon la revendication 23, 31, 32 ou 33, dans laquelle la préparation libère entre environ 70% et environ 90% du composé de formule (I) après environ 8 heures dans un test de dissolution.

38. Préparation à libération contrôlée selon la revendication 23, 31, 32 ou 33, dans laquelle la préparation libère moins d'environ 40% du composé de formule (I) au cours des 100 premières minutes dans un test de dissolution.

39. Préparation à libération contrôlée selon la revendication 23, 31, 32 ou 33, dans laquelle la préparation libère moins d'environ 65% du composé de formule (I) au cours des 300 premières minutes dans un test de dissolution.

40. Préparation à libération contrôlée selon la revendication 23, 31, 32 ou 33, dans laquelle au moins environ 50% du composé de formule (I) sont libérés au cours des 10 premières heures dans un test de dissolution.
